# EUROPEAN PATENT APPLICATION

(11) **EP 1 905 433 A1**
(43) Date of publication of application: **02.04.2008**
(21) Application number: 06781196.8
(22) Date of filing: 18.07.2006
(51) Int. Cl.: A61K 9/70, A61F 13/02, A61N 1/30

(54) **PERCUTANEOUS ABSORPTION PATCH WITH APPLICATION POSITION INDICATING FUNCTION, AND IONTOPHORESIS DEVICE**

(30) Priority: 15.07.2005 JP 2005207675
(71) Applicant: Transcu Ltd., 048580 Singapore (SG)
(72) Inventor: NAKAYAMA, Mizuo c/o TTI ellebeau Inc., Tokyo; 140-0002 (JP); MATSUMURA, Akihiko c/o TTI ellebeau Inc., Tokyo; 1400002 (JP); MATSUMURA,Takehiko c/o TTI ellebeau Inc., Tokyo; 1400002 (JP); AKIYAMA, Hidero c/o TTI ellebeau Inc., Tokyo; 1400002 (JP)
(74) Representative: Wagner, Karl H.
(86) International application number: PCT/JP2006/314183
(87) International publication number: WO 2007/010900

(57) **Abstract**

A patch (10) for transdermal absorption including a drug holding portion (18) and a backing layer (20) for introducing a drug into the body of an organism by means of transdermal absorption via the surface of the drug holding portion (18) or a membrane in contact with the drug holding portion (18) with the patch stuck to the organism B, at least part of the backing layer (20), the surface of the drug holding portion (18), and the membrane in contact with the drug holding portion (18) includes a remaining portion (23) (marker) capable of, by being switched to the side of the organism when the patch is stuck to the organism, indicating a place where the patch was stuck even after the patch is removed therefrom.

## Description

### [Technical Field]

The present invention relates to a patch for transdermal absorption for introducing a drug or the like into the body of an organism by means of transdermal absorption in a state of being stuck to the skin or mucosa of the organism and iontophoresis device.

### [Background Art]

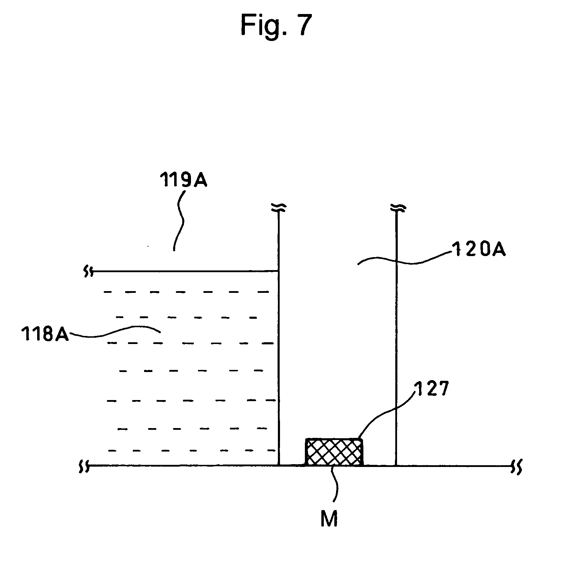

As shown in Fig. 7, the transdermal introduction of a specific substance such as a drug via a patch 1 into an organism B has been conventionally performed. In particular, in recent years, dosage means for transdermally introducing a drug into a body has been stepping into the limelight together with oral dosage and dosage by injection. This is because the dosage means solves the problems of the oral dosage and the dosage by injection. The problem of the oral dosage is that, in the course in which an administered drug reaches a target site (which is often present in the blood), the drug is decomposed in a digestive organ or the like, so the efficiency of administration is poor and hence a large amount of the drug must be administered for causing the drug to exert a sufficient drug effect. The problem of the dosage by injection is that a patient receives pain (distress) although the efficiency of administration itself is high.

In particular, as described in, for example, JP2002-532540A, a nicotine patch containing nicotine as an antismoking auxiliary agent or the like has been known to exert an effect on the administration of a drug maintaining a constant concentration over a long time period. However, such nicotine patch is desirably stuck to a position different from the place where it was stuck upon replacement for the purpose of preventing a skin stimulus peculiar to the patch from being continuously applied to a single site.

On the other hand, as shown in JP2005-510488A, a patch for transdermal absorption is often used as a local anesthetic drug using morphine hydrochloride or the like. In such case, however, the place where the patch is stuck must be properly subjected to a subsequent treatment.

As described above, it is very important to grasp the place where a patch has been stuck up to now even after the removal of the patch.

The term "patch" refers to a cloth-like section having adhesiveness on one surface mainly containing a substance such as a drug or an antigen. The structure of the patch may have some degree of thickness, or the patch may have no adhesiveness. Further, in the present invention, the term "patch" also includes that one surface thereof adhered to or contacting with an organism is constituted of a substance' face containing a drug etc. or a membrane including an ion exchange membrane etc.

Here, once a patch is removed, it is often impossible to accurately identify the site where the patch was stuck. Inparticular, for example, in the case where a patch is stuck to a part that cannot be observed by a patient himself or herself with his or her eyes, it may be difficult even to allow the patient to identify the part.

Meanwhile, the difficulty in identifying the place where a patch was stuck can be considerably alleviated by performing a treatment such as injection after the removal of the patch on condition that a person is sufficiently ready for performing the treatment immediately after the removal of the patch. However, the difficulty cannot be completely eliminated. In addition, a mental load to the person who performs the treatment is large.

In particular, in the case where a certain time interval must be placed between the removal of the patch and the next treatment (for example, the case where drug administration is performed at a certain time interval or longer), it is difficult to surely identify the preceding place where the patch was stuck.

### [Disclosure of the Invention]

The present invention has been made with a view to allowing a doctor or the like to surely perform a treatment on an effective site, and an object of the present invention is to enable the identification of the original place where a patch for transdermal absorption was stuck even after the patch is removed, the patch for transdermal absorption including a drug holding portion and a backing layer for introducing a drug or the like into the body of an organism by means of transdermal absorption in a state of being stuck to the skin, mucosa, or the like of the organism.

To solve the above problems, according to the present invention, there is provided a patch for transdermal absorption including a drug holding portion and a backing layer for introducing a drug into the body of an organism by means of transdermal absorption via the surface of the drug holding portion or a membrane in contact with the drug holding portion with the patch stuck to the organism, characterized in that at least part of the backing layer, the surface of the drug holding portion, and the membrane in contact with the drug holding portion includes a marker capable of, by being moved to the side of the organism when the patch is stuck to the organism, indicating a place where the patch was stuck even after the patch is removed therefrom.

With such constitution, it becomes possible to surely grasp the place where the patch was stuck even after the patch is removed.

In addition, the marker may be constituted as a remaining portion of at least part of the backing layer, the surface of the drug holding portion, and the membrane in contact with the drug holding portion remaining on the side of the organism even after the patch is removed therefrom.

With such constitution, it becomes possible to identify the place where the patch was stuck without introducing a substance separately serving as a marker.

In addition, the marker may be composed of a food dye or ink for printing on a tablet.

With such constitution, a marker highly safe with respect to an organism can be selected.

In addition, the patch may further include at least an electrode connected to an electric power source for actively introducing into the organism the drug in the drug holding portion by being energized.

With such constitution, a drug can be introduced into a body more quickly.

The term "marker" as used herein refers to a substance capable of, by being moved from the side of a patch to the side of an organism when the patch is stuck to the organism, indicating a place where the patch was stuck even after the patch is removed.

### [Brief Description of the Drawings]

[Fig. 1] Fig. 1 shows a patch for transdermal absorption according to an embodiment of the present invention.
[Fig. 2] Fig. 2 is a sectional view taken along the line II-II of Fig. 1.
[Fig. 3] Fig. 3 is a sectional view showing a path for transdermal absorption of an embodiment in which one surface of a drug holding portion is provided with a membrane to adhere to or contact with an organism.
[Fig. 4] Fig. 4 shows a patch for transdermal absorption having a structure with a remaining portion. (A) shows a state before the patch is stuck, (B) shows a state when the patch is stuck to an organism, (C) shows a state when the patch is removed, and (D) shows a state after the patch is removed.
[Fig. 5] A view showing a use example of the patch for transdermal absorption of Figs. 2 at the time of removal.
[Fig. 6] A schematic block diagram showing a state where the patch for transdermal absorption according to the present invention is applied as a device (patch) of an iontophoresis device.
[Fig. 7] An enlarged view of a VII portion shown in Fig. 6.
[Fig. 8] A schematic block diagram showing a state where the patch for transdermal absorption according to the present invention is applied to another iontophoresis device.
[Fig. 9] A view showing a conventional use example of a patch for transdermal absorption.

### [Best Mode for carrying out the Invention]

Hereinafter, an embodiment of the present invention will be described in detail with reference to the accompanying drawings.

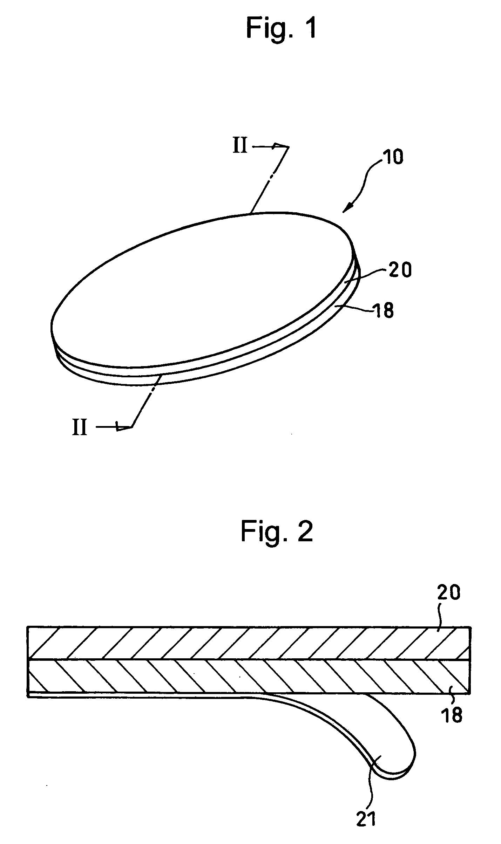

As shown in Figs. 1 and 2, in one embodiment, a patch 10 for transdermal absorption is constituted by a drug holding portion (layer) 18 holding a substance (such as a drug) to be transdermally absorbed such as an anesthetic drug and a backing layer 20 formed so as to be in close contact with the drug holding portion 18. Of those, the side of the drug holding portion 18 is a surface to be stuck to an organism. Marker (not shown) is mixed and sealed with drug in the drug holding portion 18.

The surface of the drug holding portion 18 to be stuck to the organism is provided with a film-like removable layer 21 to be removed upon sticking of the patch 10.

### (Backing layer)

The backing layer 20 functions as a primary constituent element of the patch 10, and imparts flexibility and coverage to the patch 10. A material to be used for the backing layer 20 should be inactive and should not be able to absorb the drug of a drug composition in the patch 10 or any other component such as a stabilizer. The backing layer 20 is preferably made of one or more sheets or films each made of a soft material to serve as a protective cover, to thereby allow the patch 10 to follow the shape of a skin. The backing layer 20 more preferably has air permeability (permeability for the air, vapor, and the like). A backing layer having air permeability enables the respiration (exchange between oxygen and carbon dioxide) at the site of a skin where the patch is stuck, and enables the water vapor from the surface of the skin to permeate. More specific examples of a suitable material for the backing layer include a copolyester, a polyether/polyamide copolymer, polyurethane, and a polyethylene derivative. Of course, the material for the backing layer is not limited to them. A suitable example of the polyether/polyamide copolymer includes PEBAX (registered trademark). A suitable example of polyurethane includes ESTANE. A suitable example of the polyethylene derivative includes a SKYCARE AND SCYAIR film.

### (Drug holding portion)

The drug holding portion (layer) 18 in the present embodiment is preferably, for example, a hydrogel. Any hydrogel can be suitably used for the patch 10 of the present embodiment as long as it can be subjected to γ-ray sterilization and can be impregnated with a local anesthetic drug. The hydrogel preferably has enough adhesiveness to cause the patch to adhere to an application site and is preferably able to be removed without any stimulus or damage to a wound.

The hydrogel is preferably polyvinyl pyrrolidone (PVP) crosslinked by means of an electron beam and having an average molecular weight of about 500, 000 daltons to about 2, 000, 000 daltons (more preferably about 900, 000 daltons to about 1, 500, 000 daltons). In a preferred embodiment, the hydrogel contains crosslinked polyvinyl pyrrolidone, a local anesthetic drug, and water. Of course, a stabilizer or the like can be added to the hydrogel.

A hydrogel suitably used for the patch 10 of the present embodiment is commercially available. For example, a suitable hydrogel can be purchased from Hydrogel Design Systems, or Tyco, Inc.

### (Drug or the like)

Any transdermally absorbable drug capable of exerting a therapeutic effect or a medical effect by being stuck to a patient can be used as a drug at the time of use of the patch 10 for transdermal absorption. Specific examples of such drug are listed below. Of course, the drug should not be construed as being limited to the following.

(A) A coronary vasodilator such as nitroglycerin or isosorbide nitrate

(B) A hypotensive agent such as clonidine hydrochloride or nifedipine

(C) An anesthetic painkiller such as morphine hydrochloride, lidocaine hydrochloride, or fentanyl citrate

(D) An anti-inflammatory painkiller such as ketoprofen, indomethacin, ketorolac, loxoprofen, tenidap, or buprenorfen hydrochloride

(E) A bronchodilator such as isoproterenol sulfate, salibutamol sulfate, or tulobuterol hydrochloride

(F) An androgen such as testosterone propionate or fluoxymesterone

(G) An estrogen such as estradiol benzoate, ethinylestradiol, or estriol

(H) A progesterone such as progesterone, norethisterone, or levonorgestrel

(I) An antiallergic drug such as sodium cromoglycate, azelastine, or ketotifen fumarate

(J) A muscle relaxant such as eberison hydrochloride or afloqualone

(K) An antihistamine agent such as diphenhydramine or d1-chlorpheniramine maleate

(L) A general anesthetic drug such as thiopental sodium or ketamine hydrochloride

(M) An antitussive or expectorant agent such as codeine phosphate or ephedrine hydrochloride

(N) An antismoking auxiliary agent such as nicotine

(O) A vitamin agent such as ascorbic acid

Two or more kinds of the above transdermally absorbable drugs can be used in combination as required. In addition, any one of those drugs can be sealed in the drug holding portion in the form of a compound derived to an ester body, a compound derived to an amino body, a compound derived to an acetal body, or a medically acceptable inorganic or organic salt.

### (Marker)

The marker to be used in the present embodiment must not chemically react with a drug (specific substance) to be administered to an organism and must not cause some abnormality to occur to the organism. In contrast, potential examples of the marker include various substances as long as each of them satisfies the conditions. Of course, the marker may be one that can be visually observed such as a fat dye, an aqueous dye, or an aqueous pigment. In addition, the marker may be a fluorescent dyestuff that is visualized by being irradiated with ultraviolet light, or may be a substance such as luciferin that is bound to oxygen owing to the action of a specific enzyme such as luciferase, to thereby emit fluorescence.
Furthermore, the marker may be one to be identified by means of an odor or flavor in addition to the sense of sight.

In particular, the use of a food dye as a marker is suitable from the viewpoint of safety to an organism. For example, a synthetic dyestuff such as amaranth (Food Red No. 2), erythrosine (Food Red No. 3), Allura Red AC (Food Red No. 40), new coccin (Food Red No. 102), rose bengal (Food Red No. 105), acid red (Food Red No. 106), Tartrazine (Food Yellow No. 4), sunset yellow FCF (Food Yellow No. 5), brilliant blue FCF (Food Blue No. 1), or indigocarmine (Food Blue No. 2), or a natural dyestuff such as a cochineal dyestuff or an annatto dyestuff can be widely used.

Furthermore, ink such as Opacode (registered trademark), Opacode WB (registered trademark), or NT23BR available fromColorcon to be used for printing on, for example, an oral medicine itself or a capsule of an oral medicine can also be used. This case is also suitable from the viewpoint of safety to an organism.

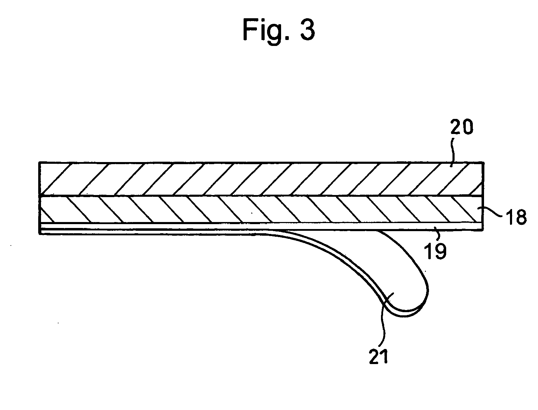

The marker may be sealed in the drug holding portion 18 while being mixed with a drug. Further, the marker mixed with the drug in the drug holding portion 18 transits into an organism B via one surface of the drug holding portion 18. However, as shown in Fig. 3, marker may transit to the organism B via a membrane 19 formed on the surface of the drug holding portion 18. Alternatively, although not shown, the marker may be introduced into a layer such as a "marker layer" separately formed by dividing a part of the drug holding portion 18.

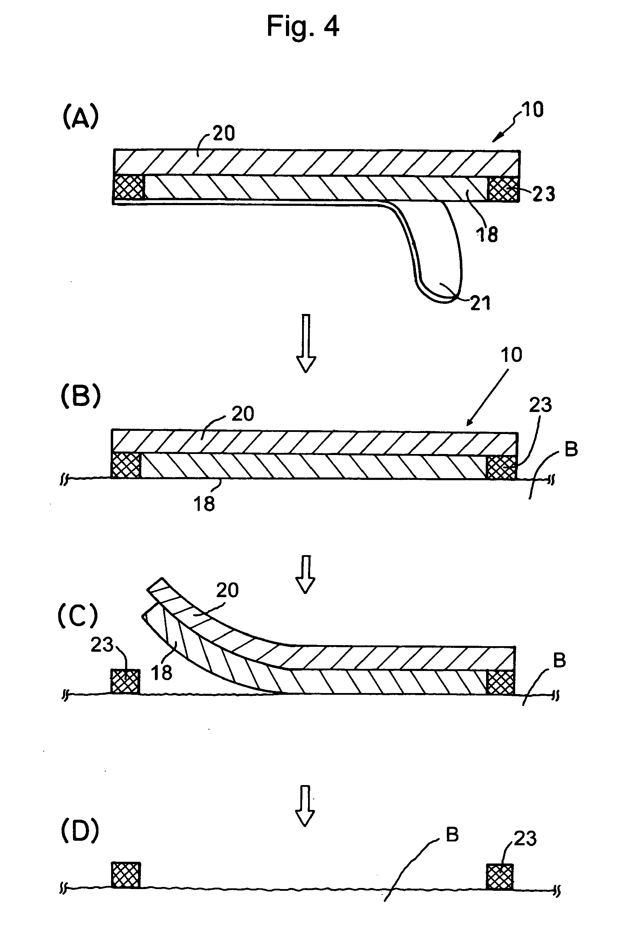

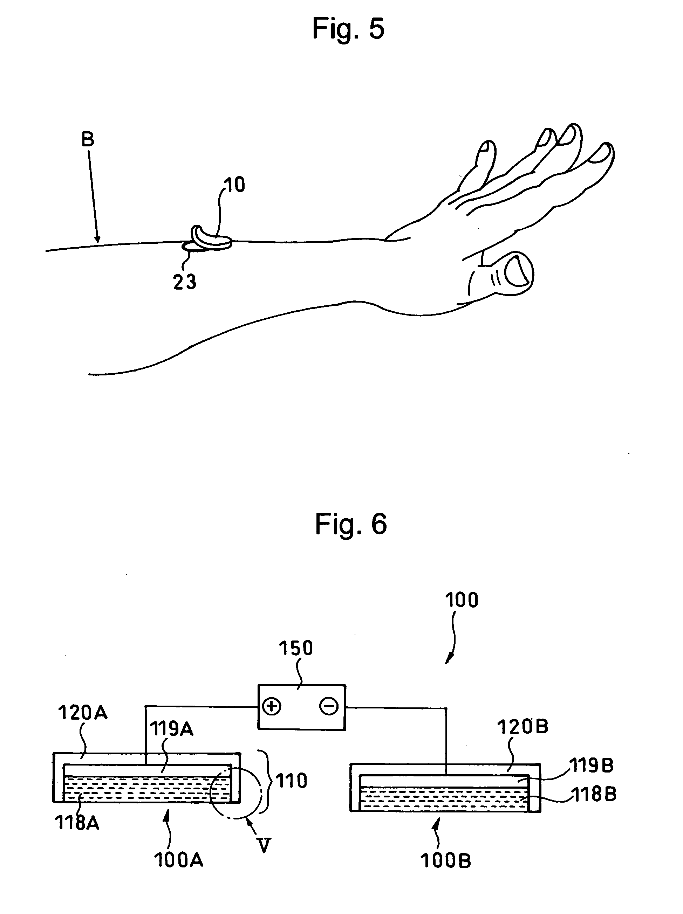

Furthermore, as shown in Fig. 4 (A), a part of the backing layer 20 may be constituted to be separable in such a manner that the part remains as a remaining portion 23 on the side of an organism when the patch 10 is removed. That is, the remaining portion 23 functions as a marker. With such constitution, a place where the patch is stuck can be identified without separate introduction of a substance to serve as a marker. Specifically, as shown in Fig. 4(B), after the patch 10 is stuck to the organism B, when the patch is removed (Fig. 4(C)), only the part as the remaining portion 23 remains on the side of the organism (Fig. 4(D)), as shown in Fig. 5 by denote 23, so the place where the patch is stuck can be clearly identified. That is, the remaining portion 23 can be remained on the organism B with such constitution that the adhesive strength between the remaining portion 23 and the side of the organism is larger than that between the remaining portion 23 and the backing layer 20 and that between the remaining portion 23 and the drug holding portion 18.

When the patch 10 for transdermal absorption is stuck to the surface of an organism (such as a skin or mucosa), the marker itself also moves to the side of the organism. Therefore, the place where the patch 10 was stuck can be accurately identified even after the patch is removed.

In addition, the use of a substance that changes its color (including becoming colorless) after the elapse of a certain time period such as a substance that changes its color when the concentration of a drug to be administered is equal to or lower than a certain concentration enables the place where the patch was stuck to be accurately identified and an accurate timing at which the patch is stuck to be identified.

More specifically, from the physiological viewpoint, the use is particularly effective for the case where a certain time interval or longer must be placed between the completion of the preceding drug administration and the next drug administration or the case where the next drug administration must be performed within a certain time period for continuously administering a drug.

Subsequently, other examples will be described in detail with reference to Figs. 6 to 8. Figs. 6 to 8 each show the application of the patch for transdermal absorption according to the present invention as a patch for an electrode for an iontophoresis device.

An iontophoresis device includes a working patch having a drug holding portion holding a drug and a non-working patch as a counter electrode of the patch, and is intended for electrically driving a drug ion by applying a voltage of the same conductivity type as that of a drug ion in the drug holding portion to the working patch in the state where both the patches are brought into contact with a skin, to thereby actively transfer the drug ion to an organism. Such iontophoresis device has advantages such as the absence of a pain to an organism, the ability to administer a drug without an initial passage effect, and the ability to electrically control the amount of a drug to be administered, so it is considered to be excellent means for administering a drug.

Fig. 6 is a sectional view schematically showing the iontophoresis device 100. Fig. 7 is an enlarged view of a V portion shown in Fig. 6.

The iontophoresis device 100 is constituted in such a manner that a working patch 100A and a ground patch (non-working patch) 100B are connected to an electric power source 150. In this example, the working patch 100A is connected to a plus side (anode), and the ground patch 100B is connected to a minus pole (cathode). For convenience of description, an iontophoresis device for administering a drug whose drug component dissociates to plus drug ions (for example, lidocaine hydrochloride as an anesthetic drug or morphine hydrochloride as an anesthetic drug) will be exemplified. An iontophoresis device for administering a drug whose drug component dissociates to minus drug ions (for example, ascorbic acid as a vitamin agent) can be constituted by reversing the polarity (plus or minus) of a voltage to be applied to an electrode and the polarity (plus or minus) of an exchange group to be introduced into an ion exchange membrane or an ion exchange resin in the following example.

As shown in Fig. 6, the working patch 100A is constituted by: an electrode 119A connected to the electric power source 150; a drug holding portion 118A holding a drug, the drug holding portion 118A being in contact with the electrode 119A to be energized from the electrode 119A; and a container 120A housing them. The ground patch 100B is constituted by: an electrode 119B; an electrolyte solution holding portion 118B holding an electrolyte solution, the electrolyte solution holding portion 118B being in contact with the electrode 119B to be energized from the electrode 119B; and a container 120B housing them. Here, the container 120A serves as a backing layer.

An electrode composed of an arbitrary conductive material can be used for each of the electrodes 119A and 119B without any limitation. An active electrode such as a silver/silver chloride couple electrode capable of suppressing the generation of an H⁺ ion or an OH⁻ ion due to the electrolysis of water or the like can also be used.

The drug holding portion 118A holds a solution (drug solution) of a drug whose drug component dissociates to plus drug ions as a result of dissolution or the like.

A marker is sealed in the drug holding portion 118A together with the drug. Of course, a divided marker holding portion (not shown) may be arranged in the drug holding portion 118A.

Furthermore, as shown in Fig. 5, the marker M may be placed in a recess 127 formed at the surface of the container 120A in contact with an organism. This holds true for an iontophoresis device 200 to be described later.

The electrolyte solution holding portion 118B holds an electrolyte solution for securing energization property. Phosphate buffer saline or physiological saline can be used as the electrolyte solution. Alternatively, the generation of a gas due to the electrolysis of water and an increase in conductive resistance or a fluctuation in pH value due to the generation can be prevented by using any one of electrolytes that are oxidized or reduced more easily than the electrolytic reaction of water (oxidation on a plus pole and reduction on a minus pole) such as: inorganic compounds such as ferrous phosphate and ferric phosphate; medical agents such as ascorbic acid (vitamin C) and sodium ascorbate; organic acids such as hydrochloric acid, oxalic acid, malic acid, succinic acid, and fumaric acid and/or salts thereof or by using a mixture of them.

Each of the drug holding portion 118A and the electrolyte solution holding portion 118B may hold a drug solution or an electrolyte solution in a liquid state. Alternatively, each of them may hold such drug solution or electrolyte solution as described above by impregnating a fibrous sheet (for example, a gauze or filter paper) or a single body made of an arbitrary material having water holding property (for example, a polymer gel sheet such as the above-described hydrogel) with the solution so that the handleability and the like of the solution can be improved.

The iontophoresis device 100 is used with the working patch 100A and the ground patch 100B each stuck to the surface of an organism.
The ground patch 100B is placed at a position on the surface (skin, mucosa, or the like) of the organism with a certain distance from the working patch 100A.

When the electric power source 150 is turned on, a plus voltage and a minus voltage are applied to the electrodes 119A and 119B, respectively. Drug ions in the drug holding portion 118A are driven by the voltages toward the organism.

A drug is actively introduced into the organism by means of the iontophoresis device in this way, whereby the drug can be more quickly absorbed and allowed to permeate into the organism.

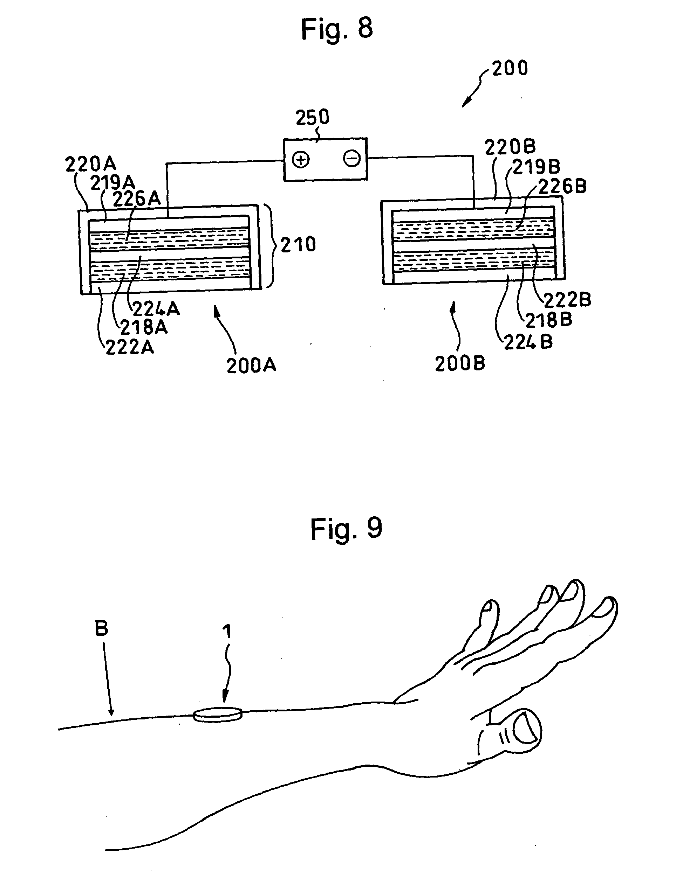

Next, anothermore iontophoresis device 200 will be described with reference to Fig. 8.

The iontophoresis device 200 is obtained by additionally developing the iontophoresis device 100. Reference numerals each having the same lower two digits as those of each of the reference numerals of the iontophoresis device 100 are given to the structure and action of the iontophoresis device 200 identical or similar to those of the iontophoresis device 100, and duplicate description is omitted.

Each of a working patch 200A and a ground patch 200B of the iontophoresis device 200 has multiple ion exchange membranes. The working patch 200A is constituted by an electrode 219A connected to an electric power source 250, a buffer solution holding portion 226A, an anion exchange membrane 224A, a drug holding portion 218A, and a cation exchange membrane 222A, and the constituents are housed in a container 220A (backing layer). The constituents come close to the surface of an organism in the stated order. On the other hand, the ground patch 200B is constituted by an electrode 219B, a buffer solution holding portion 226B, a cation exchange membrane 222A, an electrolyte solution holding portion 218B, and an anion exchange membrane 224A, and the constituents are housed in a container 220B. The constituents come close to the surface of the organism in the stated order.

Each of the anion exchange membranes 224A and 224B is an ion exchange membrane having a function of selectively passing a minus ion, and, for example, an anion exchange membrane such as a NEOSEPTA (AM-1, AM-3, AMX, AHA, ACH, or ACS) manufactured by Tokuyama Co., Ltd can be used for each of the anion exchange membranes without any particular limitation. However, each of the anion exchange membranes is not limited thereto. In addition, an anion exchange membrane of a type in which a porous film composed of a polyolefin resin, a vinyl chloride-based resin, a fluorine-based resin, a polyamide resin, a polyimide resin, or the like having cavities a part or whole of which are filled with an anion exchange resin can be particularly preferably used. In this case, the pores can be filled with the anion exchange resin by: impregnating the cavities of the porous film with a solution prepared by blending across linkable monomer such as styrene-divinylbenzene or chloromethylstyrene-divinylbenzene with a polymerization initiator; polymerizing the resultant; and introducing, into the polymer, an anion exchange group such as a primary amino group, a secondary amino group, a tertiary amino group, a quaternary ammonium group, a pyridyl group, an imidazole group, a quaternary pyridinium group, or a quaternary imidazolium group.

On the other hand, each of the cation exchange membranes 222A and 222B is an ion exchange membrane having a function of selectively passing a plus ion, and, for example, a cation exchange membrane such as a NEOSEPTA (CM-1, CM-2, CMX, CMS, or CMB) manufactured by Tokuyama Co., Ltd can be used for each of the cation exchange membranes without any particular limitation. However, each of the cation exchange membranes is not limited thereto. In addition, a cation exchange membrane of a type in which a porous film composed of a polyolefin resin, a vinyl chloride-based resin, a fluorine-based resin, a polyamide resin, a polyimide resin, or the like having cavities a part or whole of which are filled with a cation exchange resin can be particularly preferably used. In this case, the pores can be filled with the cation exchange resin by: impregnating the cavities of the porous film with a solution prepared by blending a crosslinkable monomer such as styrene-divinylbenzene or chloromethylstyrene-divinylbenzene with a polymerization initiator; polymerizing the resultant; and introducing, into the polymer, a cation exchange group such as a sulfonic group, a carboxylic group, or a phosphoric group.

In addition, in the iontophoresis device 200, the movement of a plus ion having a small molecular weight from the buffer solution holding portion 226A to the drug holding portion 218A is inhibited by the action of the anion exchange membrane 224A (the action of selectively passing a minus ion) . In addition, the movement of a minus ion having a small molecular weight from the side of the organism to the drug holding portion 218A is inhibited by the action of the cation exchange membrane (the action of selectively passing a plus ion). As a result, the decomposition of the drug at the electrode 219A and a fluctuation in pH at a skin interface are suppressed, so the stability and safety of the administration of the drug can be improved. In addition, in the iontophoresis device, the drug holding portion 218A and the buffer solution holding portion 226A are separate from each other. Therefore, the generation of a gas and a fluctuation in pH can be suppressed by: blending the electrolyte solution of each of the buffer solution holding portions 226A and 226B with a substance having an oxidation-reduction potential lower than that of water; and causing multiple ion species to be present in the buffer solution. In this case, carbon or an inactive metal such as platinum can be used for each of the electrodes 219A and 219B without a hitch. In particular, a composite carbon electrode can be used, which is constituted by: a terminal member prepared by blending a polymer matrix which has high conductivity and flexibility and which does not allow a metal ion to be eluted to transfer to an organism with a carbon powder; and a conductive sheet composed of a carbon fiber or carbon fiber paper, or a conductive sheet impregnated with a polymer elastomer.

In this example, the marker may be sealed in the drug holding portion 218A. In this case, however, the presence of the cation exchange membrane 222Amust be taken into consideration. The reason for this is as follows. If a dyestuff component of the marker dissociates to cations, the marker may be sealed in the drug holding portion 218A. If not so, the marker cannot move to the side of an organism upon sticking of the patch, so the marker cannot provide a function of indicating a place where the patch was stuck.

Accordingly, the marker can be applied to and interposed on the surface (surface in contact with the organism) of the cation exchange membrane (membrane in contact with the drug holding portion) 222A, or, although not shown, can be placed in a recess arranged on a part of the surface of the container 220A in contact with the organism instead of being sealed in the drug holding portion 218A.

With such constitution, in each of the iontophoresis devices 100 and 200, when a patch is stuck to an organism in order to perform iontophoresis, a marker moves to the side of the organism, whereby a place where the patch was stuck can be accurately identified even after the patch is removed.

### [Industrial Applicability]

The present invention is widely applicable to a patch for transdermal absorption to be used in the medical field.

## Claims

1. A patch for transdermal absorption, comprising a drug holding portion and a backing layer for introducing a drug into an organism by transdermal absorption via a surface of the drug holding portion or a membrane in contact with the drug holding portion with the patch stuck to the organism, **characterized in that**
at least one of the surface of the backing layer, the drug holding portion, and the membrane in contact with the drug holding portion is provided with a marker capable of, by being switched to a side of the organism when the patch is stuck to the organism, indicating a place where the patch was stuck even after the patch is removed therefrom.

2. The patch for transdermal absorption according to claim 1,
**characterized by** that the marker is mixed with the drug and sealed in the drug holding portion.

3. The patch for transdermal absorption according to claim 1,
**characterized by** that the marker comprises a remaining portion of at least part of the backing layer, the surface of the drug holding portion, and the membrane in contact with the drug holding portion remaining on the side of the organism even after the patch is removed therefrom.

4. The patch for transdermal absorption according to claim 1,
**characterized in that** a container is formed at the backing layer and housing the drug holding portion, a recess is formed at the surface of the container in contact with the organism and the marker is place in the recess.

5. The patch for transdermal absorption according to claim 1, 2 or 4 **characterized by** the marker comprises a food dye or ink for printing on a tablet.

6. The patch for transdermal absorption according to any one of claims 1 to 5, **characterized by** further comprising at least an electrode connected to an electric power source for actively introducing into the organism the drug in the drug holding portion by being energized.

7. An iontophoresis device, comprising working patch having a drug holding portion holding a drug and a non-working patch as a counter electrode of the patch, and is intended for electrically driving a drug ion by applying a voltage of the same conductivity type as that of a drug ion in the drug holding portion to the working patch in the state where both the patches are brought into contact with a skin, to thereby actively transfer the drug ion to an organism, wherein the iontophoresis device according to claim 5 constitutes at least one of the working patch and the non-working patch.
